(19) Europäisches Patentamt
European Patent Office
Office européen des brevets

(11) Veröffentlichungsnummer: **0 084 673**
**B1**

(12) **EUROPÄISCHE PATENTSCHRIFT**

(45) Veröffentlichungstag der Patentschrift: **02.04.86**

(51) Int. Cl.⁴: **C 07 D 285/12**, A 01 N 43/82, C 07 D 417/04

(21) Anmeldenummer: **82111948.4**

(22) Anmeldetag: **23.12.82**

(54) **Aminothiadiazole, Verfahren zu ihrer Herstellung und ihre Verwendung zur Bekämpfung unerwünschten Pflanzenwuchses.**

(30) Priorität: **22.01.82 DE 3201861**
**23.07.82 DE 3227554**

(43) Veröffentlichungstag der Anmeldung:
**03.08.83 Patentblatt 83/31**

(45) Bekanntmachung des Hinweises auf die Patenterteilung:
**02.04.86 Patentblatt 86/14**

(84) Benannte Vertragsstaaten:
**BE DE FR GB NL**

(56) Entgegenhaltungen:
**DE - A - 2 044 622**
**DE - A - 2 336 407**
**US - A - 4 218 236**
**US - A - 4 230 480**
**US - A - 4 233 057**
**US - A - 4 252 960**
**US - A - 4 252 961**
**US - A - 4 264 353**
**US - A - 4 268 675**
**US - A - 4 269 983**

**R.WEGLER, Chemie der Pflanzenschutz- und Schädlingsbekämpfungsmittel, Band 5, Springer-Verlag,**

(73) Patentinhaber: **BASF Aktiengesellschaft, Carl-Bosch-Strasse 38, D-6700 Ludwigshafen (DE)**

(72) Erfinder: **Schirmer, Ulrich, Dr., Berghalde 79, D-6900 Heidelberg (DE)**
Erfinder: **Plath, Peter, Dr., Berner Weg 24, D-6700 Ludwigshafen (DE)**
Erfinder: **Sauter, Hubert, Dr., Neckarpromenade 20, D-6800 Mannheim (DE)**
Erfinder: **Wuerzer, Bruno, Dr., Ruedigerstrasse 13, D-6701 Otterstadt (DE)**

(56) Entgegenhaltungen: (Fortsetzung)
**Berlin, Seite 440(1977) und Band 8, Seiten 3,10,12,76,208,209,(1982)**

**Die Akte enthält technische Angaben, die nach dem Eingang der Anmeldung eingereicht wurden und die nicht in dieser Patentschrift enthalten sind.**

**EP 0 084 673 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Aminothiadiazole, Verfahren zu ihrer Herstellung, Herbizide, die diese Verbindungen als Wirkstoffe enthalten, sowie ein Verfahren zur Bekämpfung unerwünschten Pflanzenwuchses mit diesen Verbindungen.

2-Amino-5-phenoxymethyl-1,3,4-thiadiazole sind als Vorprodukte für die Synthese herbizid wirksamer 3-(5-Phenoxymethyl-1,3,4-thiadiazol-2-yl-imidazolidinone bekannt (US-PS 4 252 961). Weiterhin ist bekannt, dass 2-Amino-5-phenyl(alkyl)-1,3,4-thiadiazole pharmakologisch wirksam sind (DE-OS 22 12 245; DE-OS 25 10 439; DE-OS 21 26 261). Herbizid wirksame 2-Amino-5-benzyl-1,3,4-thiadiazole werden in der DE-OS 23 36 407 beschrieben; die Wirkung ist bei diesen Verbindungen auf die Anwendung im Vorauflaufverfahren begrenzt.

Es wurde gefunden, dass Aminothiadiazole der Formel

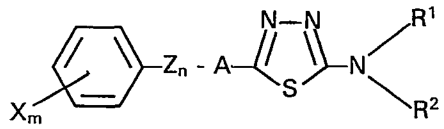

(Ia),

in der

$R^1$ und $R^2$ unabhängig voneinander Wasserstoff, $C_1$-$C_{10}$-Alkyl, $C_3$-$C_8$-Cycloalkyl, $C_2$-$C_5$-Alkoxyalkyl, Phenyl, Benzyl oder 2-Phenylethyl oder $R^1$ und $R^2$ zusammen eine gegebenenfalls durch $C_1$-$C_4$-Alkyl substituierte und gegebenenfalls Sauerstoff als Kettenglied enthaltende Alkylenkette mit bis zu 6 Kohlenstoffatomen,

A eine gegebenenfalls durch $C_1$-$C_4$-Alkyl substituierte Alkylenkette mit 1 bis 8 Kohlenstoffatomen,

Z Sauerstoff oder Schwefel,

n 0 oder 1,

X Halogen, Cyano, Nitro, $C_1$-$C_6$-Alkyl, $C_1$-$C_6$-Alkoxy, $C_1$-$C_4$-Halogenalkyl, $C_1$-$C_4$-Halogenalkoxy, $C_1$-$C_4$-Alkylthio, $C_1$-$C_4$-Alkylsulfonyl, $C_3$-$C_6$-Cycloalkyl, Phenyl, gegebenenfalls durch Halogen substituiertes Aryloxy und m 0, 1, 2, 3 oder 4 bedeuten

und in der anstelle des Restes 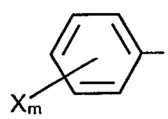

ein gegebenenfalls durch Halogen, $C_1$-$C_4$-Alkyl oder $C_1$-$C_4$-Alkoxy substituierter Naphthylrest stehen kann, mit der Massgabe, dass $R^1$ und $R^2$ nicht gleichzeitig für Wasserstoff stehen, wenn n 0 und A $-CH_2-$ bedeuten,
eine herbizide Wirkung haben und unerwünschte Pflanzen in Kulturpflanzen selektiv bekämpfen.

Aminothiadiazole der Formel

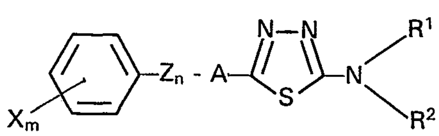

(I),

in der

$R^1$ und $R^2$ unabhängig voneinander Wasserstoff, $C_1$-$C_{10}$-Alkyl, $C_3$-$C_8$-Cycloalkyl, $C_2$-$C_5$-Alkoxyalkyl, Phenyl, Benzyl oder 2-Phenylethyl oder $R^1$ und $R^2$ zusammen eine gegebenenfalls durch $C_1$-$C_4$-Alkyl substituierte und gegebenenfalls Sauerstoff als Kettenglied enthaltende Alkylenkette mit bis zu 6 Kohlenstoffatomen,

A eine gegebenenfalls durch $C_1$-$C_4$-Alkyl substituierte Alkylenkette mit 1 bis 8 Kohlenstoffatomen,

Z Sauerstoff oder Schwefel,

n 0 oder 1,

X Halogen, Cyano, Nitro, $C_1$-$C_6$-Alkyl, $C_1$-$C_6$-Alkoxy, $C_1$-$C_4$-Halogenalkyl, $C_1$-$C_4$-Halogenalkoxy, $C_1$-$C_4$-Alkylthio, $C_1$-$C_4$-Alkylsulfonyl, $C_3$-$C_6$-Cycloalkyl, Phenyl, gegebenenfalls durch Halogen substituiertes Aryloxy und m 0, 1, 2, 3 oder 4 bedeuten

und in der anstelle des Restes 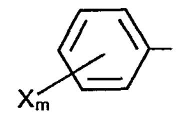

ein gegebenenfalls durch Halogen, $C_1$-$C_4$-Alkyl oder $C_1$-$C_4$-Alkoxy substituierter Naphthylrest stehen kann, mit der Massgabe, dass $R^1$ und $R^2$ nicht gleichzeitig für Wasserstoff stehen, wenn n 0 bedeutet und mit der Massgabe, dass $R^2$ nicht für Wasserstoff, n-Butyl, Allyl, Cyclohexyl oder Phenyl steht, wenn $R^1$ Wasserstoff und A gegebenenfalls durch $C_1$-$C_4$-Alkyl substituiertes Methylen bedeuten, sind neu.

Die Substituenten in Formel I bzw. Ia können die folgenden Bedeutungen haben:

$R^1$ und $R^2$ können unabhängig voneinander Wasserstoff, $C_1$-$C_{10}$-Alkyl, vorzugsweise $C_1$-$C_4$-Alkyl, wie Methyl, Ethyl, n-Propyl, i-Propyl, t-Butyl, n-Decyl, $C_3$-$C_8$-Cycloalkyl, wie Cyclopropyl, Cyclohexyl, Cyclooctyl, $C_2$-$C_5$-Alkoxyalkyl, wie Methoxyethyl, n-Propoxyethyl, Phenyl, Benzyl oder 2-Phenylethyl bedeuten. Ausserdem können sie zusammen eine gegebenenfalls durch $C_1$-$C_4$-Alkyl, vorzugsweise durch Methyl, einfach oder mehrfach substituierte und gegebenenfalls Sauerstoff als Kettenglied enthaltende Alkylenkette mit bis zu 6 Kohlenstoffatomen, wie Butylen, Pentylen, 1,4-Dimethylbutylen, 3-Oxapentylen, 2,4-Dimethyl-3-oxapentylen, bilden.

A bedeutet eine gegebenenfalls durch $C_1$-$C_4$-Alkyl substituierte Alkylenkette mit 1 bis 8 Kohlenstoffatomen, z.B. Methylen, Methylmethylen, Ethylen, Methylethylen, Dimethylmethylen, Dimethylethylen, Propylen, Methylpropylen, Ethylmethylen, Butylen, Pentylen, Hexylen, Heptylen, Methylbutylen, Octylen,

X bedeutet Halogen, wie Chlor, Brom, Fluor, Iod, Cyano, Nitro, $C_1$-$C_6$-Alkyl, $C_1$-$C_6$-Alkoxy, $C_1$-$C_4$-Alkylthio, $C_1$-$C_4$-Halogenalkyl, $C_1$-$C_4$-Halogenalkoxy, $C_1$-$C_4$-Alkylsulfonyl, $C_3$-$C_6$-Cycloalkyl, Phenyl oder gegebenenfalls durch Halogen substituiertes Aryloxy, wie gegebenenfalls durch Halogen substituiertes Phenoxy, beispielsweise Methyl, Ethyl, t-Butyl, Methoxy, Ethoxy, t-Butoxy, n-Hexyloxy, Methylthio, Ethylthio, n-Butylthio, Trifluormethyl, Difluormethyl, 1,1,2-Trifluor-2-chlorethoxy, Methylsulfonyl, Cyclopentyl, Cyclohexyl, Phenoxy, 4-Chlorphenoxy, 2,4-Dichlorphenoxy,

Z bedeutet Sauerstoff oder Schwefel.

Anstelle des Restes 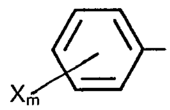

kann in Formel I auch ein gegebenenfalls durch Halogen, $C_1$-$C_4$-Alkyl oder $C_1$-$C_4$-Alkoxy substituierter α- oder β-Naphthylrest, wie α-Naphthyl, β-Naphthyl, 4-Chlor-α-naphthyl, 7-Methoxy-α-naphthyl, 2-Methyl-α-naphthyl, stehen.

Bevorzugte Aminothiadiazole der Formel Ia sind solche, bei denen $R^1$ Wasserstoff, $R^2$ $C_1$-$C_4$-Alkyl, A eine Alkylenkette mit 1 bis 4 Kohlenstoffatomen, n 0 oder 1, Z Sauerstoff, X Halogen oder $C_1$-$C_4$-Alkyl und m 1, 2 oder 3 bedeuten, oder Aminothiadiazole der Formel I, bei denen $R^1$ Wasserstoff, $R^2$ $C_1$-$C_4$-Alkyl, A eine Alkylenkette mit 2 bis 4 Kohlenstoffatomen, n 0 oder 1, Z Sauerstoff, X Halogen oder $C_1$-$C_4$-Alkyl und m 1, 2 oder 3 bedeuten.

Man erhält die Aminothiazole der Formel I durch Umsetzung von Carbonsäuren der Formel

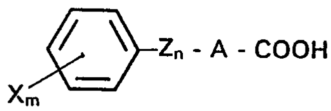 (II),

in der A, X, Z, m und n die oben genannten Bedeutungen haben, mit Thiosemicarbaziden der Formel

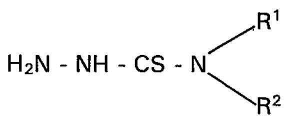 (III),

in der $R^1$ und $R^2$ die oben genannten Bedeutungen haben.

Diese Reaktion kann in Gegenwart eines unter den Reaktionsbedingungen inerten Lösungsmittels durchgeführt werden. Geeignet sind Kohlenwasserstoffe, wie Benzin, Toluol, Cyclohexan, Halogenkohlenwasserstoffe, wie Methylenchlorid, Chlorbenzol, Dichlorbenzol, oder Ether, wie Tetrahydrofuran, Dioxan. Die Reaktionstemperatur liegt im Bereich zwischen 0 und 150°C, vorzugsweise im Bereich zwischen 40 und 120°C. Zweckmässigerweise setzt man ein wasserabspaltendes Mittel, wie Schwefelsäure, Flusssäure, Polyphosphorsäure, Phosphorpentoxid oder Phosphoroxychlorid zu.

*Beispiel 1*

Zu 25 g ω-4-Chlorphenoxybuttersäure, 10,6 g Thiosemicarbazid und 250 g Dioxan wurden bei 90°C innerhalb einer halben Stunden 6 g Phosphoroxychlorid zugetropft. Dann wurde 1 Stunde lang unter Rühren bei Rückflusstemperatur gehalten, nach dem Abkühlen am Rotationsverdampfer eingeengt. Der Rückstand wurde mit Wasser und so viel Natronlauge, dass der pH sich auf etwa 10 einstellt, verrührt. Die ausgefallenen Kristalle wurden abgesaugt und getrocknet. Die so erhaltene weisse Substanz hat einen Schmelzpunkt von 188 bis 190°C und folgende Struktur:

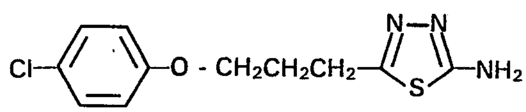

Entsprechend werden folgende Aminothiadiazole der Formel I bzw. Ia hergestellt:

| Nr. | A | $Z_n$ | $X_m$ | 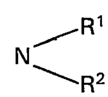 | Fp. [°C] |
|---|---|---|---|---|---|
| 2 | $-CH_2-$ | O | 4-Cl | $NHCH_3$ | 138-140 |
| 3 | $-CH_2-$ | O | 4-Cl | $NH_2$ | 215-217 |
| 4 | $-CH_2-$ | — | 4-Phenyl | $NHCH_3$ | 165-167 |
| 5 | $-CH_2-$ | — | 4-Phenyl | $NH_2$ | 229-231 |
| 6 | $-CH_2-$ | — | H | $NHCH_3$ | 117-118 |
| 7 | $-CH_2-$ | — | H | $NH_2$ | 178-180 |
| 8 | $-(CH_2)_3-$ | O | 4-Cl | $NHCH_3$ | 105-107 |
| 9 | $-(CH_2)_3-$ | O | 4-Cl | $NHC_2H_5$ | 98-99 |
| 10 | $-(CH_2)_3-$ | — | H | $NHCH_3$ | 56-58 |
| 11 | $-(CH_2)_3-$ | — | H | $NH_2$ | 180-182 |
| 12 | $-(CH_2)_4-$ | O | 4-Cl | $NHCH_3$ | 83-85 |
| 13 | $-(CH_2)_4-$ | O | 4-Cl | $NH_2$ | 157-160 |
| 14 | $-(CH_2)_2-$ | O | 4-$C_4H_9$-tert. | $NHCH_3$ | 114-116 |
| 15 | $-(CH_2)_3-$ | O | 4-$C_4H_9$-tert. | $NH_2$ | 194-196 |
| 16 | $-(CH_2)_3-$ | O | H | $NHCH_3$ | 88-90 |
| 17 | $-(CH_2)_3-$ | O | 4-$C_2H_5$ | $NH_2$ | 178-180 |
| 18 | $-(CH_2)_3-$ | O | 4-$CH_3O$ | $NH_2$ | 186-188 |
| 19 | $-(CH_2)_3-$ | O | 4-Br | $NH_2$ | 200-202 |
| 20 | $-(CH_2)_3-$ | O | 3-F | $NH_2$ | 172-174 |
| 21 | $-(CH_2)_3-$ | O | 2-Cl | $NH_2$ | 184-186 |

| Nr. | A | $Z_n$ | $X_m$ | $N(R^1)(R^2)$ | Fp. [°C] |
|---|---|---|---|---|---|
| 22 | $-(CH_2)_3-$ | O | 3,4-$Cl_2$ | $NH_2$ | 164-166 |
| 23 | $-(CH_2)_3-$ | O | 3,5-$Cl_2$ | $NH_2$ | 171-172 |
| 24 | $-(CH_2)_3-$ | O | 4-CN | $NH_2$ | 154-156 |
| 25 | $-(CH_2)_3-$ | O | 3-$CH_3$ | $NH_2$ | 180-182 |
| 26 | $-(CH_2)_3-$ | O | 4-$CH_3$ | $NH_2$ | 197-200 |
| 27 | $-(CH_2)_3-$ | O | 3-Cl | $NH_2$ | 149-150 |
| 28 | $-(CH_2)_3-$ | O | 3-$CH_3$ | $NHCH_3$ | 108-110 |
| 29 | $-(CH_2)_3-$ | O | 3-Cl | $NHCH_3$ | 85-86 |
| 30 | $-(CH_2)_3-$ | O | 4-$CH_3$ | $NHCH_3$ | 115-117 |
| 31 | $-(CH_2)_3-$ | O | 2-Cl | $NHCH_3$ | 68-70 |
| 32 | $-(CH_2)_3-$ | O | 4-$NO_2$ | $NHCH_3$ | 135-137 |
| 33 | $-(CH_2)_3-$ | O | 4-CN | $NHCH_3$ | 119-121 |
| 34 | $-(CH_2)_3-$ | O | 4-Br | $NHCH_3$ | 138-140 |
| 35 | $-(CH_2)_3-$ | O | 4-$OCH_3$ | $NHCH_3$ | 110-112 |
| 36 | $-(CH_2)_3-$ | O | 4-t-$C_4H_9$ | $NHCH_3$ | 107-109 |
| 37 | $-(CH_2)_3-$ | O | 4-$C_2H_5$ | $NHCH_3$ | 100-102 |
| 38 | $-(CH_2)_3-$ | O | 3-$CF_3$ | $NHCH_3$ | 78-80 |
| 39 | $-(CH_2)_3-$ | O | 3,5-$Cl_2$ | $NHCH_3$ | 89-90 |
| 40 | $-(CH_2)_3-$ | O | 3,4-$Cl_2$ | $NHCH_3$ | 103-105 |
| 41 | $-(CH_2)_3-$ | O | 2-$CH_3$ | $NHCH_3$ | 84-86 |
| 42 | $-(CH_2)_3-$ | O | 3-F | $NHCH_3$ | 72-74 |
| 43 | $-(CH_2)_3-$ | O | 2,4-$Cl_2$ | $NHCH_3$ | 118-120 |
| 44 | $-(CH_2)_3-$ | O | 2-$CH_3$, 4-Cl | $NHCH_3$ | 134-138 |
| 45 | $-CH(CH_3)CH_2CH_2$ | O | 4-Cl | $NHCH_3$ | 109-111 |
| 46 | $-CH(CH_3)CH_2$ | — | H | $NHCH_3$ | 83-85 |
| 47 | $-CH(CH_3)CH_2$ | — | 4-$NO_2$ | $NHCH_3$ | 132-135 |
| 48 | $-CH_2-$ | — | 4-Cl | $NHCH_3$ | 134-136 |
| 49 | $-CH_2-$ | — | 2,4-$Cl_2$ | $NHCH_3$ | 128-130 |
| 50 | $-CH_2-$ | — | 3-$CH_3$ | $NHCH_3$ | 95-96 |
| 51 | $-CH_2-$ | — | 4-$OCH_3$ | $NHCH_3$ | 124-125 |
| 52 | $-CH_2-$ | — | 3-$CF_3$ | $NHCH_3$ | 75-76 |
| 53 | $-CH_2-$ | — | 3-$OCH_3$ | $NHCH_3$ | 85-87 |
| 54 | $-CH_2-$ | — | 2-Cl | $NHCH_3$ | 102-104 |
| 55 | $-CH_2-$ | — | 2-$OCH_3$ | $NHCH_3$ | 116-117 |
| 56 | $-CH_2-$ | — | 4-$CH_3$ | $NHCH_3$ | 112-114 |
| 57 | $-CH_2-$ | — | 2-$NO_2$ | $NHCH_3$ | 68-71 |
| 58 | $-CH(C_2H_5)-$ | — | H | $NHCH_3$ | 99-100 |
| 59 | $-CH_2CH_2-$ | — | H | $NHCH_3$ | 102-104 |
| 60 | $-C(CH_3)_2CH_2-$ | — | H | $NHCH_3$ | 147-150 |
| 61 | $-C(CH_3)_2CH_2-$ | — | 4-$CH_3$ | $NHCH_3$ | 132-135 |
| 62 | $-CH_2-CH(CH_3)-$ | — | 4-t-$C_4H_9$ | $NHCH_3$ | 96-97 |
| 63 | $-CH_2-$ | O | 2,4-$Cl_2$ | $NHCH_3$ | 141-142 |
| 64 | $-CH_2-$ | O | 2-$CH_3$, 4-Cl | $NHCH_3$ | 120-122 |
| 65 | $-CH_2-$ | S | 2-$CH_3$, 4-Cl, 5-$CH_3$ | $NHCH_3$ | 155-158 |
| 66 | $-CH(CH_3)-$ | O | 2-$CH_3$, 4-Cl | $NHCH_3$ | Öl |
| 67 | $-CH_2-CH_2-$ | O | H | $NHCH_3$ | 98-101 |
| 68 | $-CH_2-CH_2-$ | O | 4-Cl | $NHCH_3$ | 114-116 |
| 69 | $-(CH_2)_3-$ | — | 4-$OCH_3$ | $NHCH_3$ | 79-81 |
| 70 | $-(CH_2)_4-$ | — | H | $NHCH_3$ | 77-79 |
| 71 | $-(CH_2)_4-$ | — | 4-$CH_3$ | $NHCH_3$ | 103-105 |
| 72 | $-(CH_2)_3-$ | O | 4-i-$C_3H_7$ | $NHCH_3$ | 74-76 |
| 73 | $-(CH_2)_4-$ | O | H | $NHCH_3$ | 98-100 |
| 74 | $-(CH_2)_2-$ | — | H | $NH_2$ | 178-180 |
| 75 | $-(CH_2)_4-$ | O | 2,4-$Cl_2$ | $NHCH_3$ | 74-76 |
| 76 | $-(CH_2)_4-$ | O | 2-$CH_3$, 4-Cl | $NHCH_3$ | 74-76 |
| 77 | $-(CH_2)_4-$ | O | 2-$CH_3$ | $NHCH_3$ | 69-71 |

| Nr. | A | $Z_n$ | $X_m$ | 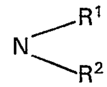 | Fp. [°C] |
|---|---|---|---|---|---|
| 78 | $-(CH_2)_4-$ | O | 3,4-$Cl_2$ | $NHCH_3$ | 80-82 |
| 79 | $-(CH_2)_4-$ | O | 2-$CH_3$ | $NH_2$ | 143-145 |
| 80 | $-(CH_2)_4-$ | O | 3,4-$Cl_2$ | $NH_2$ | 135-137 |
| 81 | $-(CH_2)_4-$ | O | 3-$CH_3$ | $NH_2$ | |
| 82 | $-(CH_2)_4-$ | O | 3-$CH_3$ | $NHCH_3$ | 70-71 |
| 83 | $-(CH_2)_4-$ | O | H | $NHC_2H_5$ | 94-96 |
| 84 | $-(CH_2)_4-$ | O | 2-$CH_3$ | $NHC_2H_5$ | 57-59 |
| 85 | $-(CH_2)_4-$ | O | 3,4-$Cl_2$ | $NHC_2H_5$ | 96-97 |
| 86 | $-(CH_2)_4-$ | O | 3-t-$C_4H_9$ | $NHCH_3$ | 58-60 |
| 87 | $-(CH_2)_5-$ | — | 4-$CH_3$ | $NHCH_3$ | 64-66 |
| 88 | $-(CH_2)_4-$ | — | H | $N(CH_3)_2$ | Öl |
| 89 | $-(CH_2)_3-$ | O | 3,4-$Cl_2$ | $N(CH_3)_2$ | 98-100 |
| 90 | $-(CH_2)_4-$ | O | 3,4-$Cl_2$ | $N(CH_3)_2$ | Öl |
| 91 | $-(CH_2)_4-$ | — | H | $N(C_2H_5)_2$ | Öl |
| 92 | $-(CH_2)_3-$ | O | 3,4-$Cl_2$ | $N(C_2H_5)_2$ | 40-42 |
| 93 | $-(CH_2)_4-$ | O | 3,4-$Cl_2$ | $N(C_2H_5)_2$ | Öl |
| 94 | $-(CH_2)_3-$ | O | 3,4-$Cl_2$ | Pyrrolidin | 117-119 |
| 95 | $-(CH_2)_4-$ | O | 3,4-$Cl_2$ | Pyrrolidin | Öl |
| 96 | $-(CH_2)_3-$ | O | 3,4-$Cl_2$ | Piperidin | 84-85 |
| 97 | $-(CH_2)_3-$ | O | 3,4-$Cl_2$ | Morpholin | 96-97 |
| 98 | $-(CH_2)_4-$ | — | H | Pyrrolidin | 68-70 |
| 99 | $-(CH_2)_4-$ | — | H | Piperidin | 45-47 |
| 100 | $-(CH_2)_4-$ | — | H | Morpholin | 62-64 |

Entsprechend können folgende Aminothiadiazole der Formel Ia erhalten werden:

| Nr. | A | $Z_n$ | $X_m$ | 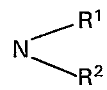 | Fp. [°C] |
|---|---|---|---|---|---|
| 101 | $-(CH_2)_4-$ | O | 2,4,5-$Cl_3$ | $NHCH_3$ | |
| 102 | $-CH_2-$ | O | 4-$NO_2$ | $NHCH_3$ | |
| 103 | $-C(CH_3)_2-$ | O | 2-$CH_3$ | $NHCH_3$ | |
| 104 | $-(CH_2)_6-$ | — | H | $NHCH_3$ | |
| 105 | $-(CH_2)_6-$ | O | H | $NHCH_3$ | |
| 106 | $-CH_2-$ | S | 4-Cl | $NHCH_3$ | |
| 107 | $-CH_2-CH_2CH(CH_3)-$ | O | 4-Cl | $NHCH_3$ | |
| 108 | $-(CH_2)_3-$ | O | 4-F | $NHCH_3$ | |
| 109 | $-(CH_2)_3-$ | O | 2-F | $NHCH_3$ | |
| 110 | $-(CH_2)_3-$ | O | 4-J | $NHCH_3$ | |
| 111 | $-(CH_2)_3-$ | O | 4-Cl | $NHC_3H_7$ | |
| 112 | $-(CH_2)_3-$ | O | 4-Cl | NH-i-$C_3H_7$ | |
| 113 | $-(CH_2)_3-$ | O | 4-Cl | $NHC_4H_9$ | |
| 114 | $-(CH_2)_3-$ | O | 4-Cl | NH-t-$C_4H_9$ | |
| 115 | $-(CH_2)_3-$ | O | 4-$CF_3$ | NH-$CH_3$ | |
| 116 | $-(CH_2)_3-$ | O | 4-$C_5H_9$ | NH-$CH_3$ | |
| 117 | $-(CH_2)_3-$ | O | 4-$C_6H_{13}$ | NH-$CH_3$ | |
| 118 | $-(CH_2)_3-$ | O | 4-(4'-Chlorphenoxy) | NH-$CH_3$ | |
| 119 | $-(CH_2)_3-$ | O | 4-(2',4'-Dichlorphenoxy) | NH-$CH_3$ | |
| 120 | $-(CH_2)_3-$ | O | 4-Phenoxy | NH-$CH_3$ | |
| 121 | $-(CH_2)_3-$ | O | 3-$OC_2H_5$ | NH-$CH_3$ | |
| 122 | $-(CH_2)_3-$ | O | 4-$OCHF_2$ | NH-$CH_3$ | |
| 123 | $-(CH_2)_3-$ | O | 3-$OCF_2CHFCl$ | NH-$CH_3$ | |
| 124 | $-(CH_2)_3-$ | O | 4-$SC_4H_9$ | NH-$CH_3$ | |
| 125 | $-(CH_2)_3-$ | O | H | $NH_2$ | |
| 126 | $-(CH_2)_2-$ | O | 4-Cl | $NH_2$ | |

| Nr. | A | $Z_n$ | $X_m$ | 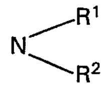 | Fp. [°C] |
|---|---|---|---|---|---|
| 127 | $-(CH_2)_2-$ | O | 4-Cl | $NHC_2H_5$ | |
| 128 | $-(CH_2)_3-$ | O | $3-NO_2$ | $NHCH_3$ | |
| 129 | $-(CH_2)_3-$ | S | H | $NHCH_3$ | |
| 130 | $-(CH_2)_3-$ | S | 4-Cl | $NHCH_3$ | |
| 131 | $-(CH_2)_3-$ | O | $4-SCH_3$ | $NHCH_3$ | |
| 132 | $-(CH_2)_3-$ | O | $4-SO_2CH_3$ | $NHCH_3$ | |
| 133 | $-CH_2-$ | — | $2-CH_3$ | $NHCH_3$ | |
| 134 | $-CH_2-$ | — | $3,5-(CH_3)_2$ | $NHCH_3$ | 108-110 |
| 135 | $-CH_2-$ | — | $3,5-Cl_2$ | $NHCH_3$ | |
| 136 | $-CH_2-CH_2-$ | — | $3,4-Cl_2$ | $NHCH_3$ | |
| 137 | $-CH_2-$ | O | $2,4,5-Cl_3$ | $NHCH_3$ | |
| 138 | -CH-<br>$CH_3$ | O | $2,4-Cl_2$ | $NHCH_3$ | |
| 139 | $-(CH_2)_3-$ | O | $2,6-(CH_3)_2$ | $NHCH_3$ | |
| 140 | $-(CH_2)_4-$ | O | $4-t-C_4H_9$ | $NHCH_3$ | |
| 141 | $-(CH_2)_4-$ | O | 3-Cl | $NHCH_3$ | 75-77 |
| 142 | $-(CH_2)_4-$ | O | 4-Br | $NHCH_3$ | 101-103 |
| 143 | $-(CH_2)_4-$ | O | 3-Br | $NHCH_3$ | 79-81 |
| 144 | $-(CH_2)_4-$ | O | $3-CF_3$ | $NHCH_3$ | |
| 145 | $-(CH_2)_4-$ | O | $3,5-(CH_3)_2$ | $NHCH_3$ | 66-67 |
| 146 | $-(CH_2)_4-$ | O | $4-OCH_3$ | $NHCH_3$ | |
| 147 | $-(CH_2)_4-$ | O | $3-OCH_3$ | $NHCH_3$ | Öl |
| 148 | $-(CH_2)_4-$ | O | $3,5-Cl_2$ | $NHCH_3$ | 65-66 |
| 149 | $-(CH_2)_4-$ | O | $2,5-Cl_2$ | $NHCH_3$ | 76-77 |
| 150 | $-(CH_2)_4-$ | O | $3-NO_2$ | $NHCH_3$ | 99-101 |
| 151 | $-(CH_2)_4-$ | O | 2-Cl | $NHCH_3$ | 77-78 |
| 152 | $-(CH_2)_4-$ | O | $2,6-(CH_3)_2$ | $NHCH_3$ | |
| 153 | $-(CH_2)_4-$ | O | $3,4-Cl_2$ | $N(i-C_3H_7)_2$ | |
| 154 | $-(CH_2)_4-$ | O | $3,4-Cl_2$ | Piperidin | |
| 155 | $-(CH_2)_4-$ | O | $3,4-Cl_2$ | Morpholin | |
| 156 | $-(CH_2)_6-$ | O | $3,4-Cl_2$ | $NHCH_3$ | 81-83 |
| 157 | $-(CH_2)_5-$ | O | $3,4-Cl_2$ | $NHCH_3$ | 84-86 |
| 158 | $-(CH_2)_7-$ | O | $3,4-Cl_2$ | $NHCH_3$ | 78-80 |
| 159 | $-(CH_2)_3-$ | O | 4-Cl | $N(CH_3)_2$ | |
| 160 | $-(CH_2)_3-$ | O | 4-Cl | $N(C_2H_5)_2$ | |
| 161 | $-(CH_2)_3-$ | O | 4-Cl | Pyrrolidin | |
| 162 | $-(CH_2)_3-$ | O | 4-Cl | Piperidin | |
| 163 | $-(CH_2)_3-$ | O | 4-Cl | Morpholin | |
| 164 | $-(CH_2)_3-$ | O | 4-Cl | NH-Phenyl | |
| 165 | $-(CH_2)_3-$ | O | 4-Cl | $N(CH_3)$-Phenyl | |
| 166 | $-(CH_2)_3-$ | O | $3,4-Cl_2$ | NH-Phenyl | 145-147 |
| 167 | $-(CH_2)_3-$ | O | $3,4-Cl_2$ | NH-Benzyl | 125-126 |
| 168 | $-(CH_2)_3-$ | O | $3,4-Cl_2$ | $NH-CH_2CH_2$-phenyl | 66-67 |
| 169 | $-(CH_2)_4-$ | O | $3,4-Cl_2$ | $NH-n-C_{10}H_{22}$ | |
| 170 | $-(CH_2)_4-$ | O | $3,4-Cl_2$ | NH-cycl.$C_6H_{11}$ | |
| 171 | $-(CH_2)_4-$ | O | $3,4-Cl_2$ | $NH-CH_2CH_2OCH_3$ | |
| 172 | $-(CH_2)_4-$ | O | $3,4-Cl_2$ | NH-cycl.$C_3H_5$ | |
| 173 | $-(CH_2)_4-$ | O | $3,4-Cl_2$ | 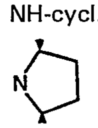 | |
| 174 | $-(CH_2)_4-$ | O | $3,4-Cl_2$ | 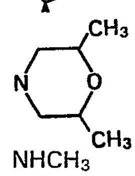 | |
| 175 | $-(CH_2)_4-$ | S | 4-Cl | $NHCH_3$ | |
| 176 | $-(CH_2)_4-$ | — | $4-CH_3$ | $N(CH_3)_2$ | |

| Nr. | A | $Z_n$ | $X_m$ | 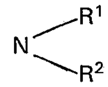 | Fp. [°C] |
|---|---|---|---|---|---|
| 177 | $-(CH_2)_4-$ | — | $4-CH_3$ | $N(C_2H_5)_2$ | |
| 178 | $-(CH_2)_4-$ | — | $4-CH_3$ | Pyrrolidin | |
| 179 | $-(CH_2)_4-$ | — | $4-CH_3$ | Piperidin | |
| 180 | $-(CH_2)_4-$ | — | $4-CH_3$ | Morpholin | |
| 181 | $-(CH_2)_3-$ | O | $4-O-nC_6H_{13}$ | $NHCH_3$ | |
| 182 | $-(CH_2)_3-$ | O | $4-cycl.C_6H_{11}$ | $NHCH_3$ | |
| 183 | $-(CH_2)_3-$ | O | 4-Phenyl | $NHCH_3$ | |
| 189 | $-(CH_2)_3-$ | O | $3,5-Cl_2$ | $N(i-C_3H_7)_2$ | 78-80 |
| 190 | $-(CH_2)_3-$ | O | $3,4-Cl_2$ | $NH(cycl-C_6H_{11})$ | 85-86 |
| 191 | $-(CH_2)_3-$ | O | $3,4-Cl_2$ | $N(CH_3)$phenyl | 85 |
| 192 | $-(CH_2)_3-$ | O | $3,4-Cl_2$ | $NH(i-C_3H_7)$ | 117-118 |
| 193 | $-(CH_2)_4-$ | O | $3,4-(CH_3)_2$ | $NHCH_3$ | 97-98 |
| 194 | $-(CH_2)_4-$ | — | 4-Cl | $NHCH_3$ | Öl |
| 195 | $-(CH_2)_4-$ | — | $4-OCH_3$ | $NHCH_3$ | Öl |
| 196 | $-(CH_2)_4-$ | — | $3,4-Cl_2$ | $NHCH_3$ | |
| 197 | $-(CH_2)_4-$ | — | $3,5-Cl_2$ | $NHCH_3$ | |
| 198 | $-(CH_2)_5-$ | — | 4-Cl | $NHCH_3$ | 172-175 |

| Nr. | A | $Z_n$ | 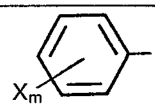 | 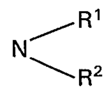 | Fp. [°C] |
|---|---|---|---|---|---|
| 184 | $-(CH_2)_3-$ | O | α-Naphthyl | $NHCH_3$ | 108-110 |
| 185 | $-(CH_2)_3-$ | O | β-Naphthyl | $NHCH_3$ | 114-116 |
| 186 | $-(CH_2)_3-$ | O | β-Naphthyl | $NH_2$ | 176-178 |
| 187 | $-(CH_2)_4-$ | O | α-Naphthyl | $NHCH_3$ | 105-107 |
| 188 | $-(CH_2)_4-$ | O | β-Naphthyl | $NHCH_3$ | 123-125 |

Die Verbindungen der Formel I bzw. Ia können beispielsweise in Form von direkt versprühbaren Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wässrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100 000 Gewichtsteilen Wasser erhält man eine wässrige Dispersion, die 0,02 Gewichtsprozent des Wirkstoffs enthält.

III. 20 Gewichtsteile der Verbindung Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

IV. 20 Gewichtsteile der Verbindung Nr. 8 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

V. 20 Gewichtsteile der Verbindung Nr. 7 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 6 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 51 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 1 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (postdirected, lay-by).

Die Aufwandmengen an Wirkstoff betragen je nach Jahreszeit, Bekämpfungsziel und Wachstumsstadium 0,1 bis 5 kg/ha und mehr, vorzugsweise 1 bis 3 kg/ha.

Die herbizide Wirkung von Verbindungen der Formel I bzw. Ia wird durch Gewächshausversuche gezeigt.

Als Kulturgefässe dienen Plastikblumentöpfe mit 300 $cm^3$ Inhalt und lehmigem Sand mit etwa 1,5% Humus als Substrat. Die Samen der Testpflanzen werden nach Arten getrennt flach eingesät. Unmittelbar danach werden die Wirkstoffe bei Vorauflaufbehandlung auf die Erdoberfläche aufgebracht. Sie werden hierzu in Wasser als Verteilungsmittel suspendiert oder emulgiert und mittels fein verteilender Düsen gespritzt. Die Aufwandmengen betragen 3,0

kg Wirkstoff/ha. Nach dem Aufbringen der Mittel werden die Gefässe leicht beregnet, um Keimung und Wachstum in Gang zu bringen. Danach deckt man die Gefässe mit durchsichtigen Plastikhauben ab, bis die Pflanzen angewachsen sind. Diese Abdeckung bewirkt ein gleichmässiges Keimen der Testpflanzen, sofern diese nicht durch die Wirkstoffe beeinträchtigt werden.

Zum Zwecke der Nachauflaufbehandlung zieht man die Testpflanzen je nach Wuchsform bis zu einer Wuchshöhe von 3 bis 15 cm an und behandelt sie danach. Zur Nachauflaufbehandlung werden entweder direkt gesäte und in den gleichen Gefässen aufgewachsene Pflanzen ausgewählt, oder sie werden erst als Keimpflanzen getrennt angezogen und einige Tage vor der Behandlung in die Versuchsgefässe verpflanzt. Die Aufwandmengen für die Nachauflaufbehandlung variieren je nach Wirkstoff. Sie betragen 1,0, 2,0 oder 3,0 kg Wirkstoff/ha. Die Abdeckung unterbleibt bei der Nachauflaufbehandlung.

Die Versuchsgefässe werden im Gewächshaus aufgestellt, wobei für wärmeliebende Arten wärmere Bereiche (20 bis 35°C) und für solche gemässigter Klimate 10 bis 20°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Die in den Versuchen verwendeten Pflanzen setzen sich aus folgenden Arten zusammen:

| | |
|---|---|
| Amaranthus spp. | (Fuchsschwanz), |
| Beta vulgaris | (Zuckerrüben), |
| Chenopodium album | (Weisser Gänsefuss), |
| Ipomea spp. | (Prunkwindearten), |
| Galium aparine | (Kletten-Labkraut), |
| Lolium multiflorum | (Ital. Raygras), |
| Sinapis alba | (Weisser Senf), |
| Solanum nigrum | (Schwarzer Nachtschatten), |
| Triticum aestivum | (Weizen), |
| Urtica urens | (Kleine Brennessel), |
| Viola tricolor | (Stiefmütterchen). |

Bei Vorauflaufanwendung von 3,0 kg Wirkstoff/ha im Gewächshaus zeigt beispielsweise die Verbindung Nr. 50 eine gute herbizide Aktivität.

Bei Nachauflaufanwendung von 3,0 kg Wirkstoff/ha bekämpfne beispielsweise die Verbindungen Nr. 41, 64, 75, 77 und 101 breitblättrige Pflanzen. Die Verbindungen Nr. 77 und 78 bekämpfen auch bei niedrigeren Aufwandmengen beispielhaft ausgewählte unerwünschte breitblättrige Pflanzen, ohne die Kulturpflanzen zu schädigen.

In Anbetracht der Vielseitigkeit der Applikationsmethoden können die erfindungsgemässen Verbindungen bzw. die erfindungsgemässen herbiziden Mittel noch in einer weiteren Zahl von Kulturpflanzen zur Beseitigung unerwünschter Pflanzen eingesetzt werden.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuss |
| Asparagus officinalis | Spargel |
| Avena sativa | Hafer |
| Beta vulgaris spp. altissima | Zuckerrübe |
| Beta vulgaris spp. rapa | Futterrübe |
| Brassica napus var. napobrassica | Kohlrübe |
| Brassica napus var. rapa | Weisse Rübe |
| Camellia sinensis | Teestrauch |
| Carthamus tinctorius | Saflor - Färberdistel |
| Carya illinoinensis | Pekannussbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süsskartoffeln |
| Juglans regia | Walnussbaum |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europea | Ölbaum |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weisstanne |
| Pinus spp. | Kiefer |
| Pisum sativum | Gartenerbse |
| Prunus avium | Süsskirsche |
| Prunus domestica | Pflaume |
| Prunus dulcis | Mandelbaum |

| Botanischer Name | Deutscher Name |
|---|---|
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preisselbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die erfindungsgemässen Verbindungen bzw. die erfindungsgemässen herbiziden Mittel mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Ausserdem kann es von Nutzen sein, die Verbindungen der Formel I bzw. Ia allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

**Patentansprüche**

1. Aminothiadiazole der Formel

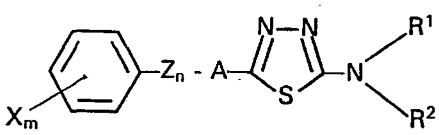

(I),

in der

$R^1$ und $R^2$ unabhängig voneinander Wasserstoff, $C_1$-$C_{10}$-Alkyl, $C_3$-$C_8$-Cycloalkyl, $C_2$-$C_5$-Alkoxyalkyl, Phenyl, Benzyl oder 2-Phenylethyl oder $R^1$ und $R^2$ zusammen eine gegebenenfalls durch $C_1$-$C_4$-Alkyl substituierte und gegebenenfalls Sauerstoff als Kettenglied enthaltende Alkylenkette mit bis zu 6 Kohlenstoffatomen,

A eine gegebenenfalls durch $C_1$-$C_4$-Alkyl substituierte Alkylenkette mit 1 bis 8 Kohlenstoffatomen,

Z Sauerstoff oder Schwefel,

n 0 oder 1,

X Halogen, Cyano, Nitro, $C_1$-$C_6$-Alkyl, $C_1$-$C_6$-Alkoxy, $C_1$-$C_4$-Halogenalkyl, $C_1$-$C_4$-Halogenalkoxy, $C_1$-$C_4$-Alkylthio, $C_1$-$C_4$-Alkylsulfonyl, $C_3$-$C_6$-Cycloalkyl, Phenyl, gegebenenfalls durch Halogen substituiertes Aryloxy und

m 0, 1, 2, 3 oder 4 bedeuten

und in der anstelle des Restes 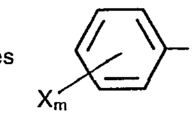

ein gegebenenfalls durch Halogen, $C_1$-$C_4$-Alkyl oder $C_1$-$C_4$-Alkoxy substituierter Naphthylrest stehen kann,

mit der Massgabe, dass $R^1$ und $R^2$ nicht gleichzeitig für Wasserstoff stehen, wenn n 0 bedeutet und mit der Massgabe, dass $R^2$ nicht für Wasserstoff, n-Butyl, Allyl, Cyclohexyl oder Phenyl steht, wenn $R^1$ Wasserstoff und A gegebenenfalls durch $C_1$-$C_4$-Alkyl substituiertes Methylen bedeuten.

2. Aminothiadiazole der Formel I gemäss Anspruch 1, dadurch gekennzeichnet, dass

$R^1$ Wasserstoff, $R^2$ $C_1$-$C_4$-Alkyl,

A eine Alkylenkette mit 2 bis 4 Kohlenstoffatomen,

n 0 oder 1,

Z Sauerstoff,

X Halogen oder $C_1$-$C_4$-Alkyl und

m 1, 2 oder 3 bedeuten.

3. Verfahren zur Herstellung von Aminothiadiazolen der Formel I gemäss Anspruch 1, dadurch gekennzeichnet, dass man Carbonsäuren der Formel

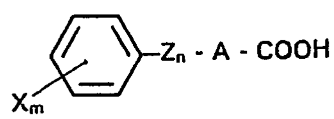

(II),

in der A, X, Z, m und n die im Anspruch 1 genannten Bedeutungen haben,

mit Thiosemicarbaziden der Formel

$$H_2N - NH - CS - NR^1R^2 \qquad (III),$$

in der $R^1$ und $R^2$ die im Anspruch 1 genannten Bedeutungen haben,

in Gegenwart eines unter den Reaktionsbedingungen inerten Lösungsmittels umsetzt.

4. Herbizid, enthaltend ein Aminothiadiazol der Formel I gemäss Anspruch 1.

5. Herbizid nach Anspruch 4, dadurch gekennzeichnet, dass es ein Aminothiadiazol der Formel I enthält, wobei

$R^1$ Wasserstoff, $R^2$ $C_1$-$C_4$-Alkyl,

A eine Alkylenkette mit 2 bis 4 Kohlenstoffatomen,

n 0 oder 1, Z Sauerstoff,

X Halogen oder $C_1$-$C_4$-Alkyl und

m 1, 2 oder 3 bedeuten.

6. Herbizid, enthaltend inerte Zusatzstoffe und ein Aminothiadiazol der Formel

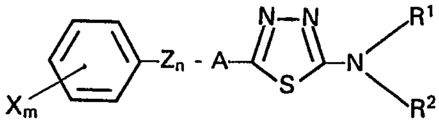
(Ia),

in der

$R^1$ und $R^2$ unabhängig voneinander Wasserstoff, $C_1$-$C_{10}$-Alkyl, $C_3$-$C_8$-Cycloalkyl, $C_2$-$C_5$-Alkoxyalkyl, Phenyl, Benzyl oder 2-Phenylethyl oder $R^1$ und $R^2$ zusammen eine gegebenenfalls durch $C_1$-$C_4$-Alkyl substituierte und gegebenenfalls Sauerstoff als Kettenglied enthaltende Alkylenkette mit bis zu 6 Kohlenstoffatomen,

A eine gegebenenfalls durch $C_1$-$C_4$-Alkyl substituierte Alkylenkette mit 1 bis 8 Kohlenstoffatomen,

Z Sauerstoff oder Schwefel,

n 0 oder 1,

X Halogen, Cyano, Nitro, $C_1$-$C_6$-Alkyl, $C_1$-$C_6$-Alkoxy, $C_1$-$C_4$-Halogenalkyl, $C_1$-$C_4$-Halogenalkoxy, $C_1$-$C_4$-Alkylthio, $C_1$-$C_4$-Alkylsulfonyl, $C_3$-$C_6$-Cycloalkyl, Phenyl, gegebenenfalls durch Halogen substituiertes Aryloxy und

m 0, 1, 2, 3 oder 4 bedeuten

und in der anstelle des Restes 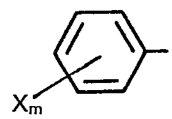

ein gegebenenfalls durch Halogen, $C_1$-$C_4$-Alkyl oder $C_1$-$C_4$-Alkoxy substituierter Naphthylrest stehen kann,

mit der Massgabe, dass $R^1$ und $R^2$ nicht gleichzeitig für Wasserstoff stehen, wenn n 0 und A $-CH_2-$ bedeuten.

7. Herbizid nach Anspruch 6, dadurch gekennzeichnet, dass es ein Aminothiadiazol der Formel Ia enthält, wobei

$R^1$ Wasserstoff, $R^2$ $C_1$-$C_4$-Alkyl,

A eine Alkylenkette mit 1 bis 4 Kohlenstoffatomen,

n 0 oder 1, Z Sauerstoff,

X Halogen oder $C_1$-$C_4$-Alkyl und

m 1, 2 oder 3 bedeuten.

8. Verfahren zur Bekämpfung unerwünschten Pflanzenwuchses, dadurch gekennzeichnet, dass man eine herbizid wirksame Menge eines Aminothiadiazols der Formel I gemäss Anspruch 1 auf die Pflanzen und/oder ihren Standort einwirken lässt.

9. Verfahren zur Bekämpfung unerwünschten Pflanzenwuchses, dadurch gekennzeichnet, dass man eine herbizid wirksame Menge eines Aminothiadiazols der Formel Ia gemäss Anspruch 6 auf die Pflanzen und/oder ihren Standort einwirken lässt.

## Claims

1. An aminothiadiazole of the formula

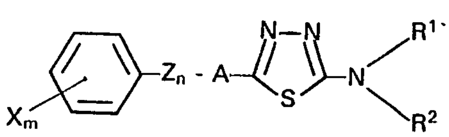
(I),

where

$R^1$ and $R^2$ independently of one another are hydrogen, $C_1$-$C_{10}$-alkyl, $C_3$-$C_8$-cycloalkyl, $C_2$-$C_5$-alkoxyalkyl, phenyl, benzyl or 2-phenylethyl, or $R^1$ and $R^2$ together form an alkylene chain of up to 6 carbon atoms which is unsubstituted or substituted by $C_1$-$C_4$-alkyl and may or may not contain oxygen as a chain member,

A is an alkylene chain of 1 to 8 carbon atoms which is unsubstituted or substituted by $C_1$-$C_4$-alkyl,

Z is oxygen or sulfur,

n is 0 or 1,

X is halogen, cyano, nitro, $C_1$-$C_6$-alkyl, $C_1$-$C_6$-alkoxy, $C_1$-$C_4$-haloalkyl, $C_1$-$C_4$-haloalkoxy, $C_1$-$C_4$-alkylthio, $C_1$-$C_4$-alkylsulfonyl, $C_3$-$C_6$-cycloalkyl, phenyl or unsubstituted or halogen-substituted aryloxy, and

m is 0, 1, 2, 3 or 4,

and where the radical 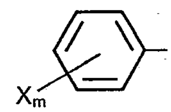

can be replaced by naphthyl which is unsubstituted or substituted by halogen, $C_1$-$C_4$-alkyl or $C_1$-$C_4$-alkoxy,

with the proviso that $R^1$ and $R^2$ are not both hydrogen when n is 0, and that $R^2$ is not hydrogen, n-butyl, allyl, cyclohexyl or phenyl when $R^1$ is hydrogen and A is methylene which is unsubstituted or substituted by $C_1$-$C_4$-alkyl.

2. An aminothiadiazole of the formula I as claimed in claim 1, where

$R^1$ is hydrogen, $R^2$ is $C_1$-$C_4$-alkyl,

A is an alkylene chain of 2 to 4 carbon atoms,

n is 0 or 1,

Z is oxygen,

X is halogen or $C_1$-$C_4$-alkyl, and

m is 1, 2 or 3.

3. A process for the preparation of aminothiadiazoles of the formula I as claimed in claim 1, wherein a carboxylic acid of the formula

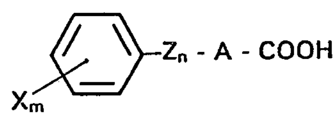
(II),

where A, X, Z, m and n have the meanings given in claim 1, is reacted with a thiosemicarbazide of the formula

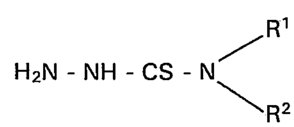
(III),

where $R^1$ and $R^2$ have the meanings given in claim 1, in the presence of a solvent which is inert under the reaction conditions.

4. A herbicide containing an aminothiadiazole of the formula I as claimed in claim 1.

5. A herbicide as claimed in claim 4, which contains an aminothiadiazole of the formula I where

$R^1$ is hydrogen, $R^2$ is $C_1$-$C_4$-alkyl,

A is an alkylene chain of 2 to 4 carbon atoms,
n is 0 or 1,
Z is oxygen,
X is halogen or $C_1$-$C_4$-alkyl, and
m is 1, 2 or 3.

6. A herbicide containing inert additives and an aminothiadiazole of the formula

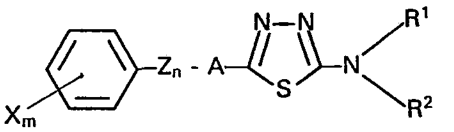

(Ia),

where
$R^1$ and $R^2$ independently of one another are hydrogen, $C_1$-$C_{10}$-alkyl, $C_3$-$C_8$-cycloalkyl, $C_2$-$C_5$-alkoxyalkyl, phenyl, benzyl or 2-phenylethyl, or $R^1$ and $R^2$ together form an alkylene chain of up to 6 carbon atoms which is unsubstituted or substituted by $C_1$-$C_4$-alkyl and may or may not contain oxygen as a chain member,
A is an alkylene chain of 1 to 8 carbon atoms which is unsubstituted or substituted by $C_1$-$C_4$-alkyl,
Z is oxygen or sulfur,
n is 0 or 1,
X is halogen, cyano, nitro, $C_1$-$C_6$-alkyl, $C_1$-$C_6$-alkoxy, $C_1$-$C_4$-haloalkyl, $C_1$-$C_4$-haloalkoxy, $C_1$-$C_4$-alkylthio, $C_1$-$C_4$-alkylsulfonyl, $C_3$-$C_6$-cycloalkyl, phenyl or unsubstituted or halogen-substituted aryloxy, and
m is 0, 1, 2, 3 or 4,

and where the radical 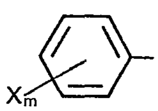

can be replaced by naphthyl which is unsubstituted or substituted by halogen, $C_1$-$C_4$-alkyl or $C_1$-$C_4$-alkoxy, with the proviso that $R^1$ and $R^2$ are not both hydrogen when n is 0 and A is $-CH_2-$.

7. A herbicide as claimed in claim 6, which contains an aminothiadiazole of the formula Ia where
$R^1$ is hydrogen, $R^2$ is $C_1$-$C_4$-alkyl,
A is an alkylene chain of 1 to 4 carbon atoms,
n is 0 or 1,
Z is oxygen,
X is halogen or $C_1$-$C_4$-alkyl, and
m is 1, 2 or 3.

8. A process for combating unwanted plant growth, wherein a herbicidally effective amount of an aminothiadiazole of the formula I as claimed in claim 1 is allowed to act on the plants and/or their location.

9. A process for combating unwanted plant growth, wherein a herbicidally effective amount of an aminothiadiazole of the formula Ia as claimed in claim 6 is allowed to act on the plants and/or their location.

**Revendications**

1. Aminothiadiazoles de la formule

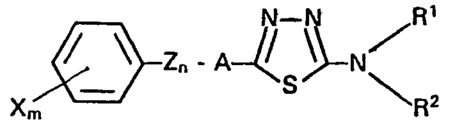

(I),

dans laquelle
$R^1$ et $R^2$ désignent chacun, indépendamment de l'autre, un atome d'hydrogène ou un groupe alkyle en $C_1$ à $C_{10}$, cycloalkyle en $C_3$ à $C_8$, alcoxy-alkyle en $C_2$ à $C_5$, phényle, benzyle ou 2-phényl-éthyle ou forment ensemble une chaîne alkylène comprenant jusqu'à six atomes de carbone et éventuellement de l'oxygène comme chaînon et éventuellement alkyl-(en $C_1$ à $C_4$)-substituée,
A représente une chaîne alkylène en $C_1$ à $C_8$, éventuellement alkyl(en $C_1$ à $C_4$)-substituée,
Z représente de l'oxygène ou du soufre,
n vaut 0 ou 1,
X désigne un atome d'halogène ou un groupe cyano, nitro, alkyle en $C_1$ à $C_6$, alcoxy en $C_1$ à $C_6$, halo-alkyle en $C_1$ à $C_4$, halo-alcoxy en $C_1$ à $C_4$, alkyl-(en $C_1$ à $C_4$)-thio, alkyl(en $C_1$ à $C_4$)-sulfonyle, cyclo-alkyle en $C_3$ à $C_6$ ou phényle ou un groupe aryl-oxy éventuellement halo-substitué et
m vaut 0, 1, 2, 3 ou 4,

le reste 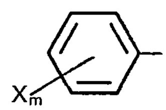

pouvant être remplacé par un groupe naphtyle éventuellement halo-, alkyl(en $C_1$ à $C_4$)- ou alcoxy(en $C_1$ à $C_4$)-substitué,
avec la condition que, lorsque n = 0, $R^1$ et $R^2$ ne peuvent pas être simultanément des atomes d'hydrogène, et la condition que, lorsque $R^1$ = H et A représente une chaîne méthylène éventuellement alkyl(en $C_1$ à $C_4$)-substituée, $R^2$ ne peut pas désigner un atome d'hydrogène, ni un groupe n-butyle, allyle, cyclohexyle ou phényle.

2. Aminothiadiazoles de la formule I selon la revendication 1, caractérisées en ce que
$R^1$ = hydrogène
$R^2$ = alkyle en $C_1$ à $C_4$
A = chaîne alkylène en $C_2$ à $C_4$
n = 0 ou 1
Z = oxygène
X = halogène ou alkyle en $C_1$ à $C_4$ et
m vaut 1, 2 ou 3.

3. Procédé de préparation d'aminothiadiazoles de la formule I selon la revendication 1, caractérisé en ce que l'on fait réagir un acide carboxylique de la formule

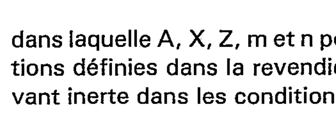

(II),

dans laquelle A, X, Z, m et n possèdent les significations définies dans la revendication 1, dans un solvant inerte dans les conditions réactionnelles, avec un thio-semicarbazide de la formule

$$H_2N - NH - CS - NR^1R^2 \qquad (III),$$

dans laquelle $R^1$ et $R^2$ possèdent les significations définies dans la revendication 1.

4. Composition herbicide, contenant un aminothiadiazole de la formule I selon la revendication 1.

5. Composition herbicide suivant la revendication 4, caractérisée en ce qu'elle contient un aminothiadiazole de la formule I, dans laquelle

$R^1$ = hydrogène
$R^2$ = alkyle en $C_1$ à $C_4$
A = chaîne alkylène en $C_2$ à $C_4$
n = 0 ou 1
Z = oxygène
X = halogène ou alkyle en $C_1$ à $C_4$ et
m vaut 1, 2 ou 3.

6. Composition herbicide, contenant, dans des additifs inertes, un aminothiadiazole de la formule

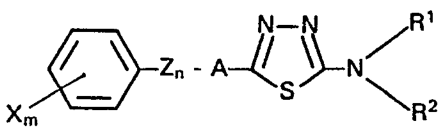 (Ia),

dans laquelle

$R^1$ et $R^2$ désignent chacun, indépendamment de l'autre, un atome d'hydrogène ou un groupe alkyle en $C_1$ à $C_{10}$, cycloalkyle en $C_3$ à $C_8$, alcoxy-alkyle en $C_2$ à $C_5$, phényle, benzyle ou 2- phényl-éthyle ou forment ensemble une chaîne alkylène comprenant jusqu'à six atomes de carbone et éventuellement de l'oxygène comme chaînon et éventuellement alkyl(en $C_1$ à $C_4$)-substituée,

A représente une chaîne alkylène en $C_1$ à $C_8$, éventuellement alkyl(en $C_1$ à $C_4$)-substituée,

Z représente de l'oxygène ou du soufre,

n vaut 0 ou 1,

X désigne un atome d'halogène ou un groupe cyano, nitro, alkyle en $C_1$ à $C_6$, alcoxy en $C_1$ à $C_6$, halo-alkyle en $C_1$ à $C_4$, halo-alcoxy en $C_1$ à $C_4$, alkyl(en $C_1$ à $C_4$)-thio, alkyl(en $C_1$ à $C_4$)-sulfonyle, cycloalkyle en $C_3$ à $C_6$ ou phényle ou un groupe aryl-oxy éventuellement halo-substitué et

m vaut 0, 1, 2, 3 ou 4,

le reste 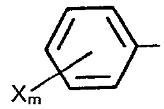

pouvant être remplacé par un groupe naphtyle éventuellement halo-, alkyl(en $C_1$ à $C_4$)- ou alcoxy(en $C_1$ à $C_4$)-substitué,

avec la condition que, lorsque n = 0 et A = $-CH_2-$, $R^1$ et $R^2$ ne peuvent pas désigner simultanément des atomes d'hydrogène.

7. Composition herbicide suivant la revendication 6, caractérisée en ce qu'elle contient un aminothiadiazole de la formule Ia, dans laquelle

$R^1$ = H
$R^2$ = alkyle en $C_1$ à $C_4$
A = chaîne alkylène en $C_1$ à $C_4$
n = 0 ou 1
Z = oxygène
X = halogène ou alkyle en $C_1$ à $C_4$ et
m vaut 1, 2 ou 3.

8. Procédé pour combattre le développement de plantes indésirables, caractérisé en ce que l'on laisse agir sur les plantes et(ou) sur leur biotope une quantité efficace du point de vue herbicide d'un aminothiadiazole de la formule I selon la revendication 1.

9. Procédé pour combattre le développement de plantes indésirables, caractérisé en ce que l'on laisse agir sur les plantes et(ou) sur leur biotope une quantité efficace du point de vue herbicide d'un aminothiadiazole de la formule Ia selon la revendication 6.